(19) Europäisches Patentamt
European Patent Office
Office européen des brevets

(11) Publication number: **0 488 965 B1**

# (12) EUROPEAN PATENT SPECIFICATION

(45) Date of publication of patent specification: **06.09.95**

(51) Int. Cl.[6]: **C07D 307/88**, A61K 31/365, C07D 493/04

(21) Application number: **91830506.1**

(22) Date of filing: **19.11.91**

## (54) Phthalidilidene derivatives of carnitine and alkanoylcarnitines and pharmaceutical compositions containing them.

(30) Priority: **20.11.90 IT 4849490**

(43) Date of publication of application:
**03.06.92 Bulletin 92/23**

(45) Publication of the grant of the patent:
**06.09.95 Bulletin 95/36**

(84) Designated Contracting States:
**AT BE CH DE DK ES FR GB GR IT LI LU NL SE**

(56) References cited:
**EP-A- 0 442 850**

**CHEMICAL AND PHARMACEUTICAL BULLETIN. vol. 31, no. 8, 1983, TOKYO JP pages 2698 - 2707; F. SAKAMOTO ET AL.: 'Studies on Prodrugs. I. Preparation and Characterization of Acyloxyallylester of Ampicillin'**

**CHEMICAL ABSTRACTS, vol. 89, no. 13, 25 September 1978, Columbus, Ohio, US; abstract no. 105410Z, T. T. NGO ET AL.: 'The inhibition of human plasma acetylcholinesterase by some naturally occurring compounds' page 579 ;column 1 ;**

**CHEMICAL ABSTRACTS, vol. 95, no. 17, 26 October 1981, Columbus, Ohio, US; abstract no. 146016T, L. GARBIN ET AL.: '"In vitro" inhibition of erythrocytes acetylcholinesterase by carnitine isomers' page 242 ;column 2 ;**

(73) Proprietor: **Sigma-Tau Industrie Farmaceutiche Riunite S.p.A.
Viale Shakespeare, 47
I-00144 Roma (IT)**

(72) Inventor: **Picciola, Giampaolo
6, Piazzale S. Baracco
I-20123 Milano (IT)**

(74) Representative: **Fassi, Aldo
c/o Sigma-Tau
Industrie Farmaceutiche Riunite S.p.A.,
Viale Shakespeare 47
I-00144 Rome (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 488 965 B1

Rank Xerox (UK) Business Services
(3.10/3.09/3.3.3)

## Description

The present invention relates to (3-phthalidylidene) alkyl esters of carnitine and alkanoyl carnitines of general formula (I)

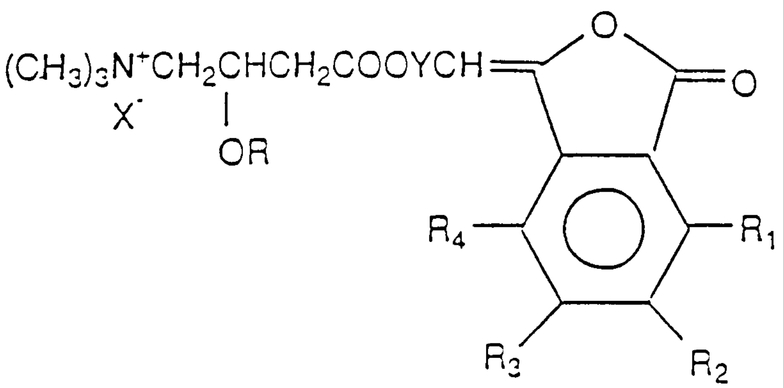

(I)

wherein

| | |
|---|---|
| Y | is a $C_1$-$C_5$ alkylene group, unsubstituted or substituted with one or more lower $C_1$-$C_4$ alkyl groups; |
| R | is hydrogen or $C_2$-$C_6$ alkanoyl; |
| $R_1$, $R_2$,$R_3$ and $R_4$, | identical or different, are hydrogen, halogen, $C_1$-$C_8$ alkoxy, lower $C_1$-$C_4$ alkyl, halogen-substituted lower alkyl, amino, alkyl-substituted amino wherein the alkyl group has 1 to 4 carbon atoms, nitro, cyano, $C_1$-$C_4$ alkanoylamino, or $R_1$ and $R_2$ taken together, $R_2$ and $R_3$ taken together or R3 and R4 taken together form a $C_1$-$C_4$ alkylenedioxy group, and |
| $X^-$ | is the anion of a pharmacologically acceptable salt. |

The esters of general formula (I) are potent PAF (plateletactivating factor) and acetylcholinesterase antagonists.

PAF is an endogenous phospholipid discovered in the early sixties, and was thus termed after its remarkable ability to aggregate platelets. PAF does not play exclusively a role in stimulating the platelets to aggregate, but also plays an important role in various pathological conditions, such as inflammatory and allergic conditions.

The esters (I) can be used as active ingredients in pharmaceutical compositions for treating those pathological conditions wherein PAF is the etiological agent or one of the etiological agents. Moreover, because of their acetylcholinesterase antagonism, they are effective for the treatment of pre-senile and senile dementias (typically, Alzheimer's disease).

In particular, in formula (I) the various substituents can have the following meanings:

| | |
|---|---|
| Y | is $C_1$-$C_2$ alkylene; |
| R | is selected from hydrogen, acetyl, propionyl, butyryl, isobutyryl, valeryl and isovaleryl; |
| $R_1$, $R_2$, $R_3$ and $R_4$, | identical or different, are selected from hydrogen, fluorine, chlorine, methoxy, ethoxy, methyl, trifluoromethyl, trichloromethyl, amino, nitro, cyano and acetylamino; |
| $X^-$ | is the anion of a pharmacologically acceptable acid selected from chloride; bromide; iodide; aspartate; particularly acid aspartate; citrate, particularly acid citrate; phosphate, particularly acid phosphate; fumarate; particularly acid fumarate; glycerophosphate; glucosephosphate; lactate; maleate; particularly acid maleate; orotate; oxalate; particularly acid oxalate, sulphate, particularly acid sulphate; trichloroacetate, trifluoroacetate and metansulphonate. |

PAF (1-0-alkyl-2-acetylglycero-3-phosphorylcholine) has formula

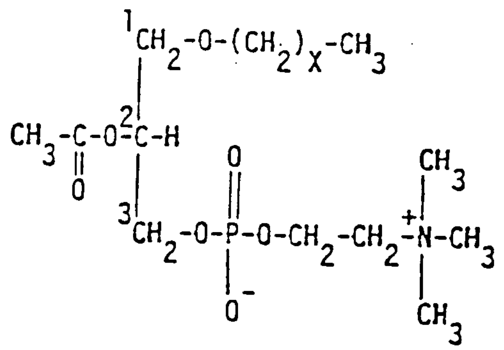

Platelet-Activating Factor (X = 15 to 17)

PAF actually represents a family of phospholipids, because the alkyl group at position 1 can vary in lenght from $C_{16}$ to $C_{18}$. PAF is biosynthesized following a stimulus by platelets, neutrophils, monocytes, mast cells, eosinophils, renal mesangial cells, renal medullary cells and vascular endothelial cells.

A recent thorough review of PAF biochemistry, pharmacology, pathobiology, receptors and antagonists has been published by P. Braquet et al. in Pharmacological reviews, vol. 39, n. 2, 97-145 (1987).

It has been definetly ascertained that PAF is involved with a variety of physiophatological conditions, including artherial thrombosis, acute inflammation, endotoxic shock, acute allergic diseases and asthma.

It is a potent bronchoconstrictor and promotes the accumulation of eosinophils in the lung, it causes tracheal and bronchial edema and it stimulates the secretion of mucus. All this has prompted remarkable interest in the development of PAF-antagonists.

The compounds of the present invention are prepared by condensing carnitine or the appropriate alkanoylcarnitine with the suitable 3-(ω-bromo-alkylidene) phtalide, optionally substituted at positions 4,5,6 and 7 with $R_4$, $R_3$, $R_2$, $R_1$, respectively, the meanings of which have been previously defined.
3-(ω-bromoalkylidene) phthalides and their preparation are described in the prior art. For instance, in the Japanese patent application 85814/1978/publication 13221/1980) to Kanebo and in Chem. Pharm. Bull. 31 -(8), 2698-2707 (1983), Fumio Sakamoto et al disclose the synthesis of (Z)- and (E)-3-(2-bromoethylidene) phthalides which these Authors use as intermediates for preparing penicillin (3-phtalidylidene) ethyl esters.

These latter compounds are prodrugs of orally administrable penicillins, which are advantageously endowed with better bioavailability with respect to the antibiotics as such.
(See also in this regard the Japanese patent application 106650/1978, publication n. 33444/1980 to Kanebo).

The synthesis described by the foregoing Authors comprises (see the following reaction scheme) the bromination of known phthalides (I) (German Auslegeschrift 1.266.310; S.N. Chakravati and W.N. Perkin, J. Chem. Soc. 1927, 196) with N-bromo-succinimide (NBS) in carbontetrachloride to give the bromides (2) which with triphenylphosphine in benzene give the phosphonium bromides (3). These latter compounds, reacted with acetaldehyde in the presence of triethylamine, give the ethylidenephtalides (4) and (5), i.e. both (Z) and (E) isomers.

By bromination of (4) and (5) with NBS, 3-(2-bromo-ethylidene) phthalides (6) in the (Z) isomer form only are obtained.

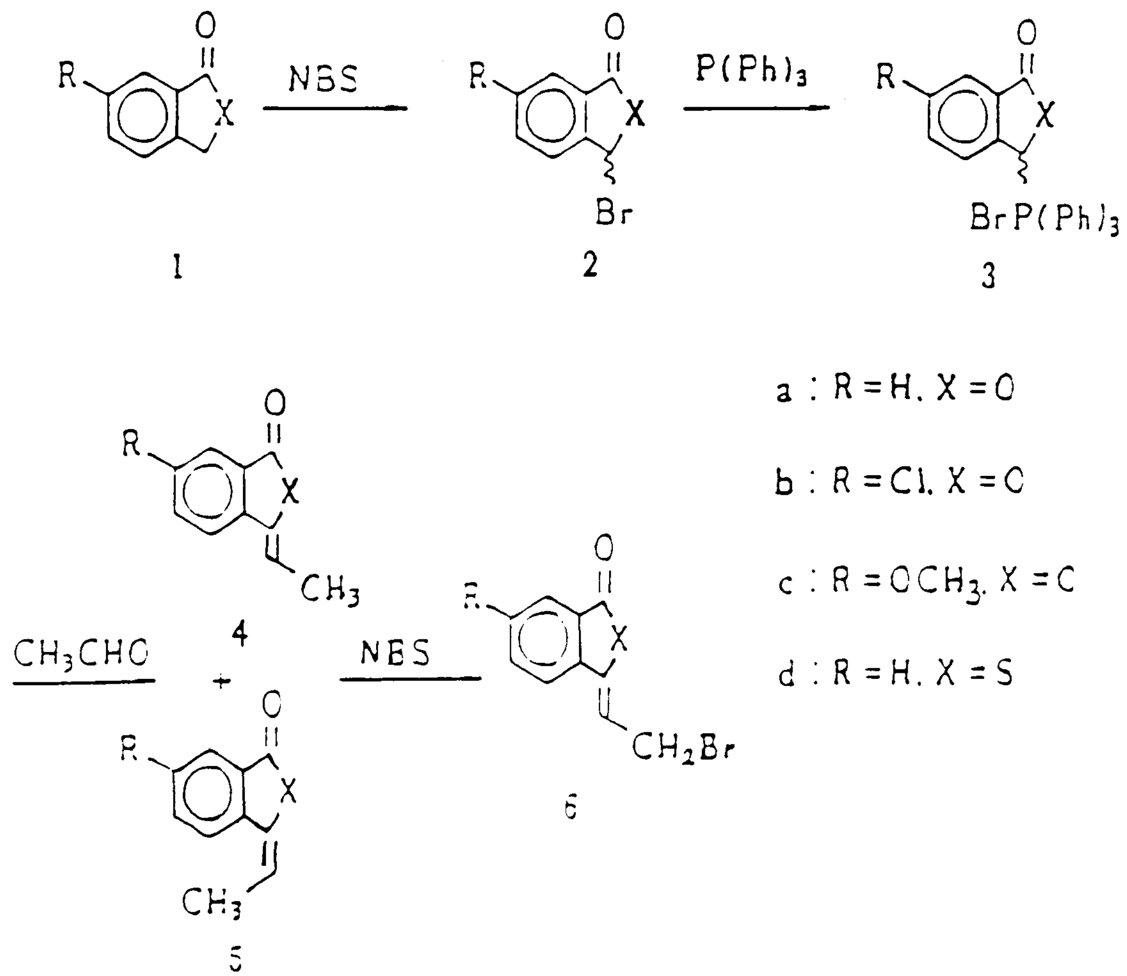


The following non-limiting examples illustrate the preparation of some of the compounds of the present invention.

Example 1

(Z)-(3-phthalidylidene)ethyl ester of L-carnitine bromide (ST 736).

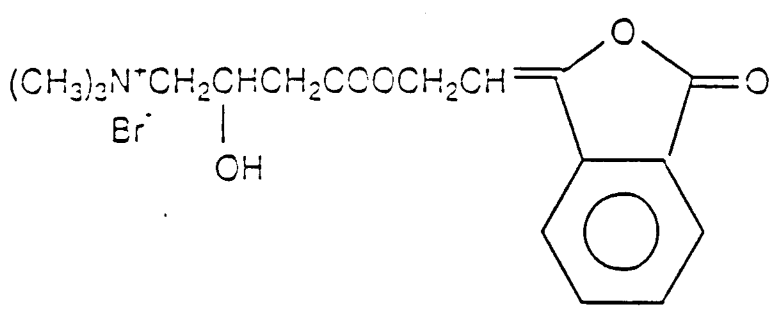

To a suspension of 3.98 g(0.0247 moles) L-carnitine (M.W. 161.2) in 30 mL dimethylformamide a solution of 5.9 g (0.0247 moles) (Z)-3-(2-bromoethylidene)phthalide (M.W. 239.07) (Chem. Pharm. Bull. 31 - (8), 2698-2707, 1983) in 30 mL dimethylformamide was added, while keeping the temperature of the reaction mixture at +25°C.

The reaction was monitored via TLC (eluant $CHCl_3$: MeOH:$H_2O$:iso-PrOH:AcOH 60:40:15:10:15).

After stirring for 3 hours at 25 °C, the very thin suspension was filtered and the solution slowly added dropwise to 800 mL ethyl ether.

The suspension that formed was stirred for 2 hours and then allowed to stand at +5 °C overnight.

The precipitate was filtered and crystallized from isopropanol.

7.44 g (3-phthalidylidene)ethyl ester of L-carnitine bromide were obtained. Yield 75.2%.

M.P. = 154-156 °C (da isoPrOH)

$[\alpha]_D^{25}$ = -10.7 ° (c = 1 in $H_2O$)

TLC: single spot

(eluant $CHCl_3$:MeOH:$H_2O$:isoPrOH:AcOH 60:40:15:10:15)

silica gel plates 0.25 mm - 60 $F_{254}$ (E. Merck)

Detectors: UV 0.254 nm and iodine vapors

ELEMENTARY ANALYSIS ($C_{17}H_{22}BrNO_5$)

| | | | | |
|---|---|---|---|---|
| Calc. % | C 51.01 | H 5.54 | Br 19.96 | N 3.50 |
| Found % | C 51.24 | H 5.67 | Br 19.72 | N 3.41 |

HPLC:

Waters 990 Column: μBondapack $C_{18}$, inner diameter = 3.9 mm, length 300 mm;

mobile phase: $KH_2PO_4$ 0.05 M/$CH_3CN$ 70:30; t = 25 °C;

flow rate: 1 ml/min; detector: UV λ = 205 nm; capacity factor (K′) calculated on the peak of $Br^-$ anion: K′ = 1.36.

$^1$H-NMR:

Varian 300 MHz($D_2$0) δ (p.p.m.):2.95-2.70(2H,m,C$\underline{H_2}$COOR),
3.385(9H,s,+N(C$\underline{H_3}$)$_3$),
3.55(2H,d,C$\underline{H_2}$N),4.90-4.80(1H,m,C$\underline{H}$OH),
4.953(2H,d,$J_{AX}$ = 7.2Hz,C$\underline{H_2}$CH =),
5.806(1H,t,$J_{AX}$ = 7.2Hz,$CH_2$C$\underline{H}$ =),7.40-7.70(4H,m,Arom)

Attribution of configuration to the C = C double bound

The (Z) configuration of the compound to the C = C double bound was attributed on the grounds of the accumulated ($CD_3CN$) $^1$H-NMR N.O.E. spectra (Nuclear Overhauser Effect) and the proton-coupled carbon spectra.

The experiment $^1$H-NMR N.O.E. (Nuclear Overhauser Effect) was conducted by irradiating the triplet at δ 6.0 p.p.m.; a doublet at δ 7.932 and 7.907 p.p.m. (J = 8.3 Hz) corresponding to C-H, was observed.

Examples 2-31

The compounds of the examples 2-31 were prepared by following the procedures outlined in Example 1 and effecting the required substitutions which, however, are apparent to anyone having ordinary skill in organic syntheses.

The formula of these compounds is obtained by substituting in the general formula (I) the meanings of R and $R_2$ given in the following table ($R_1$ = $R_3$ = $R_4$ = H, unless otherwise indicated). In the same table, the main physico-chemical characteristics of the obtained compounds are shown.

TABLE 1

| Comp. | R | $R_2$ | M.P. °C | $[\alpha]_D^{25}$ (C = 1 % $H_2O$) | yield % | HPLC K' | $^1$H-NMR Varian 300 MHz ($D_2O$) δ (p.p.m.) | ELEMENTARY ANALYSIS |
|---|---|---|---|---|---|---|---|---|
| ST 739 Ex. 2 | $COCH_3$ | H | 158 - 160°C (dec.) | - 11,6 | 68 | 2,35 | 2.164 (3 H, s, $COCH_3$) 2.97 (2 H, d, $CH_2COOR$) 3.280(9 H,s, $^+N(CH_3)_3$) 3.76-4.03 (2H, m, $CH_2N$) 4.94 (2 H, d, $CH_2CH=$) 5.74 (1H,m, CHOR) 5.83(1 H, t, $CH_2CH=$) 7.54-7.73(4 H, m, Arom). | $C_{19}H_{24}BrNO_6$<br>C H Br N<br>calc% 51,59 5,47 18,07 3,17<br>fou.% 51,38 5,39 17,99 3,13 |
| ST 768 Ex. 3 | $COCH_2CH_3$ | H | 133-135°C | - 13, 5 | 89 | 4,56 | 1.08 (3 H,m, $CH_2CH_3$) 2.42-2.48(2 H, m, $CH_2CH_3$) 2.96-2.99(2 H, m, $CH_2COOR$) 3.311 (9 H, s, $^+N(CH_3)_3$)3.80-4.03 (2 H, m, $CH_2N$) 4.92 (2 H, d, $CH_2CH=$) 5.75 (1 H, m, CHOR) 5.85 (1 H, t, $CH_2CH=$) 7.55-7.70(4 H, m, Arom). | $C_{20}H_{26}BrNO_6$<br>C H Br N<br>calc% 52,64 5,74 17,51 3,07<br>fou. % 52,62 5,83 17,45 3,03 |
| ST 775 Ex. 4 | 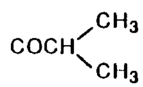 | H | 154-157°C (dec.) | - 13,5 | 87 | 6,66 | 1.10-1.13 (6 H, d, $CH(CH_3)_2$) 2.61--2.67 (1 H, m, $CH(CH_3)_2$) 2.94-3.01(2 H, m, $CH_2COOR$) 3.306 (9 H,s, $^+N(CH_3)_3$)3.82-4.06 (2 H, m, $CH_2N$) 4.93(2 H, d, $CH_2CH=$) 5.79-5.86 (2H, m, CHOR, $CH_2CH=$) 7.58-7.72 (4 H, m, Arom). | $C_{21}H_{28}BrNO_6$<br>C H Br N<br>calc% 53,62 6,00 16,99 2,98<br>fou.% 53,74 6,09 19,91 2,84 |
| ST 776 Ex. 5 | 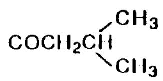 | H | 158-159°C (dec.) | - 12,1 | 67 | 11,05 | 0.84 (6 H, t,$CH(CH_3)_2$)1.92-1.97 (1H, m, $CH(CH_3)_2$) 2.26 (2 H,d, $CH_2CH(CH_3)_2$)2.95-3.03 (2 H, m, $CH_2COOR$)3.320(9 H, s, $^+N(CH_3)_3$)3.84-4.05 (2 H, m, $CH_2N$) 4.95 (2 H, d, $CH_2CH=$) 5.80-5.87(2H,m,CHOR,$CH_2CH=$) 7.60-7.78 (4 H, m, Arom). | $C_{22}H_{30}BrNO_6$<br>C H Br N<br>calc% 54,55 6,24 16,50 2,89<br>fou.% 54,40 6,27 16,34 2,79 |
| ST 801 Ex. 6 | H | Cl | 157-158°C | - 10,1 | 62,2 | 1,82 | 2.84(2 H, m, $CH_2COOR$) 3.334(9 H, s, $^+N(CH_3)_3$)3.61(2 H, d, $CH_2N$) 4.8 (1 H, m, CHOR ) 5.03 (2 H, d, $CH_2CH=$) 5.95 (1 H, t, $CH_2CH=$) 7.61-7.64 (3 H, m, Arom). | $C_{17}H_{21}BrClNO_5$<br>C H Br Cl N<br>calc% 46,97 4,87 18,38 8,16 3,22<br>fou.% 54,40 6,27 16,34 8,11 3,18 |
| ST 804 Ex 7 | $COCH_3$ | Cl | 120°C (dec.) | - 9,4 | 74,6 | 3,03 | 2.203 (3 H, s, $COCH_3$) 3.00(2 H, d, $CH_2COOR$) 3.326(9 H, s, $^+N(CH_3)_3$)3.81-4.05 (2 H, m, $CH_2N$) 5.00(2 H, d, $CH_2CH=$) 5.7 7(1 H, m, CHOR) 5.94 (1 H, t, $CH_2CH=$) 7.57-7.65 (3 H, m, Arom). | $C_{19}H_{23}BrClNO_6$<br>C H Br Cl N<br>calc% 47,87 4,86 17,76 7,44 2,94<br>fou.% 47,64 4,95 17,54 7,31 2,95 |
| ST 805 Ex. 8 | $COCH_2CH_3$ | Cl | 120-122°C | - 11,6 | 74 | 4,26 | 1.121 (3H, t, $CH_2CH_3$)2.45-2.55(2H,m,$CH_2CH_3$)2.95-3.11(2H,m, $CH_2COOR$)3.358 (9H,s,$^+N(CH_3)_3$)3.87-4.12(2H,m,$CH_2N$)5.00(2H,d, $CH_2CH=$)5.83(1H,m,CHOR)5.95(1H,t,CH=)7.54-7.70(3H,m,Arom.). | $C_{20}H_{25}BrClNO_6$<br>C H Br Cl N<br>calc% 48,95 5,13 16,28 7,22 2,85<br>fou.% 48,81 5,03 16,17 7,29 2,79 |
| ST 806 Ex 9 | 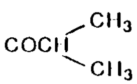 | Cl | 138-140°C | - 9 | 66 | 5,88 | 1.14-1.18(6H,m,$CH(CH_3)_2$)2.67-2.72(1H,m,$CH(CH_3)_2$),2.96-3.12 (2H,m,$CH_2COOR$)3.364(9H,s,$^+N(CH_3)_3$)3.90-4.14(2H,m,$CH_2N$)5.00 (2H,m,$CH_2CH=$)5.84(1H,m,CHOR)5.96(1H,t,$CH_2CH=$)7.55-7.73(9H, m,Arom.). | $C_{21}H_{27}BrClNO_6$<br>C H Br Cl N<br>calc% 49,97 5,39 15,83 7,02 2,77<br>fou.% 49,71 5,27 15,71 7,13 2,82 |
| ST 803 Ex. 10 | 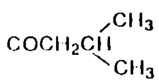 | Cl | 128-130°C | -9,2 | 70 | 8,78 | 0.85(6H,t,$CH(CH_3)_2$)2.0(1H,m,$CH(CH_3)_2$)2.3(2H,d,$CH_2CH(CH_3)_2$) 3.0(2H,m,$CH_2COOR$)3.2(9H,s,$^+N(CH_3)_3$)3.9-4.1(2H,m,$CH_2N$), 5.0(2H,d,$CH_2CH=$) 5.8(1H,m, CHOR)6.0(1H,t,$CH_2CH=$) 7.55-7.80 (3H m Arom.). | $C_{22}H_{29}BrClNO_6$<br>C H Br Cl N<br>calc% 50,93 5,63 15,40 6,83 2,70<br>fou.% 50,73 5,54 15,38 6,91 2,68 |

TABLE 1 (continuation)

| Comp. | R | $R_2$ | M.P. °C | $[\alpha]_D^{25}$ (C = 1 %$H_2O$) | yield % | HPLC K' | $^1$H-NMR Varian 300 MHz ($D_2O$) δ (p.p.m.) | ELEMENTARY ANALYSIS |
|---|---|---|---|---|---|---|---|---|
| ST 866 Ex.11 | H | $OCH_3$ | 123-126°C | -9.2 | 90 | 1.86 | 2.82 (2 H, m,$CH_2COOR$)3,33 (9 H, s,$^+N(CH_3)_3$) 3.60(2 H,m, $CH_2N^+$) 3.82 (3 H, s, $OCH_3$)4,8(1H,m,CHOH)4.93 (2H,d,$CH_2$-CH=)5,66(1 H, t $CH_2$-CH=) 6.89-7.08-7.38 (3 H, s,d,d, Arom). | $C_{18}H_{24}BrNO_6$<br>C H Br N<br>calc% 50.07 5.66 18.51 3.24<br>fou. % 49.98 5.71 18.43 3.15 |
| ST 871 Ex.12 | $COCH_3$ | $OCH_3$ | 157-158°C | -10.6 | 89 | 3.73 | 2.17(3H,s,$COCH_3$)2.97(2H,m,$CH_2COOR$)3.28(9H,s,$^+N(CH_3)_3$) 3.84(3H,s,$OCH_3$)3.78-3.99(2H,m,$CH_2N^+$) 4.91(2H,d,$CH_2$CH=)5.65(1H,t,$CH_2$-CH=) 5.74(1H,m,CHOR)6.96-7.12-7.42(3H,s,d,d,Arom) | $C_{20}H_{26}BrNO_7$<br>C H Br N<br>calc% 50.86 5.55 16.92 2.97<br>fou. % 50.77 5.50 16.88 2.83 |
| ST 869 Ex.13 | $COCH_2CH_3$ | $OCH_3$ | 132-135°C | - 13.2 | 88 | 5.95 | 1.08(3H,s,$COCH_2CH_3$)2.46(2H,q,$COCH_2CH_3$) 2.97(2H,m,$CH_2COOR$)3.29(9H,s,$^+N(CH_3)_3$) 3.83(3H,s,$OCH_3$)3.80-4.01(2H,m,$CH_2N^+$) 4.90(2H,d,$CH_2$-CH=)5.64(1H,t,$CH_2$-CH=) 5.78(1H,m,CHOR)6.95-7.12-7.42(3H,s,d,d,Arom) | $C_{21}H_{28}BrNO_7$<br>C H Br N<br>calc% 51.86 5.80 16.43 2.88<br>fou. % 51.80 5.83 16.37 2.79 |
| ST 870 Ex.14 | 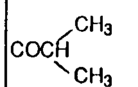 | $OCH_3$ | 145-148°C | -14.1 | 88 | 9.39 | 1.13(6H,m,$CH(CH_3)_2$)2.66(1H,m,$CH(CH_3)_2$)2.98 (2H,d,$CH_2COOR$)3.30(9H,s,$^+N(CH_3)_3$)3.84(3H,s,$OCH_3$) 3.8-4.05(2H,m,$CH_2N^+$)4.9(2H,d,$CH_2$-CH=)5.63 (1H,t,$CH_2$-CH=)5.79(1H,m,CHOR)6.97-7.13-7.43 (2H,s,d,d,Arom) | $C_{22}H_{30}BrNO_7$<br>C H Br N<br>calc% 52.81 6.04 15.97 2.80<br>fou. % 52.75 6.05 15.77 2.76 |
| ST 867 Ex.15 | 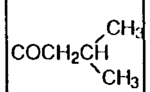 | $OCH_3$ | 140-142°C | -11.2 | 86 | 16.37 | 0.86(6H,m,$CH(CH_3)_2$)1.96(1H,m,$CH(CH_3)_2$) 2.28(2H,d,$CH_2CH(CH_3)_2$)3.99(2H,m,$CH_2COOR$) 3.33(9H,s,$^+N(CH_3)_3$)3.84(3H,s,$OCH_3$) 3.8-4.05(2H,m,$CH_2N^+$)4.91(2H,d,$CH_2$-CH=) 5.67(1H,t,$CH_2$-CH=)5.80(1H,m,CHOR)7.01-7.17--7.50 (3H,s,d,d,Arom) | $C_{23}H_{32}BrNO_7$<br>C H Br N<br>calc% 53.70 6.27 15.53 2.72<br>fou. % 53.66 6.29 15.37 2.65 |

TABLE 1 (continuation)

| Comp. | R | $R_2$ | M.P. °C | $[\alpha]^{25}_{D}$ (C = 1 %$H_2O$) | yield% | HPLC* K' | $^1$H-NMR Varian 300 MHz ($D_2O$) δ (p.p.m.) | ELEMENTARY ANALYSIS |
|---|---|---|---|---|---|---|---|---|
| ST 917 Ex.16 | H | F | 175-176°C | - 10.2 | 65 | 1.61 | 2.83 (2 H, m,$CH_2$ COOR)3,33 (9 H, s,$^+N(CH_3)_3$) 3.61(2 H,d, $CH_2N^+$) 4,8(1H,m,CHOH)4.98(2H,d,$CH_2$-CH=)5,87 (1 H, t $CH_2$-CH=) 7.40-7.70(3 H, m, Arom). | $C_{17}H_{21}BrFNO_5$<br>C H Br N<br>calc% 48.82 5.06 19.10 3.35<br>fou.% 48.64 5.02 19.30 3.43 |
| ST 931 Ex.17 | $COCH_3$ | F | 89-90°C | -9.6 (c = 0,7% $H_2O$) | 75 | 3.22 | 2.18(3H,s,$COCH_3$)2.98(2H,d,$CH_2COOR$)3.27(9H,s,$^+N(CH_3)_3$) 3.76-4.04(2H,m,$CH_2N^+$)4.98(2H,d,$CH_2$CH=)5.74(1H, m,CHOR)5.88(1H,t,$CH_2$-CH=)7.46-7.76(3H,m,Arom) | $C_{19}H_{23}BrFNO_6$<br>C H Br N<br>calc% 49.58 5.04 17.36 3.04<br>fou.% 49.74 5.02 17.40 3.01 |
| ST 926 Ex.18 | $COCH_2CH_3$ | F | 110-112°C | - 12.8 | 75 | 1.59 | 1.03(3H,t,$CH_2CH_3$)2.38-2.45(2H,m,$CH_2CH_3$)2.93-2.95 (2H,m,$CH_2COOR$)3.22(9H,s,$^+N(CH_3)_3$)3.71-3.99(2H,m, $CH_2N^+$)5.01(2H,d,$CH_2$-CH=)5.71-5.78(1H,m,CHOR)5.93 (1H,t,$CH_2$-CH=)7.53-7.84(3H,m,Arom) | $C_{20}H_{25}BrFNO_6$<br>C H Br N<br>calc% 50.64 5.31 16.85 2.95<br>fou.% 50.70 5.36 16.88 3.02 |
| ST 933 Ex.19 | 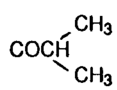 | F | 98-99°C | -11.3 | 77 | 13.45 | 1.10(6H,d,$CH(CH_3)_2$)2.58-2.68(1H,m,$CHCH_3)_2$)2.94-2.98 (2H,m,$CH_2COOR$)3.25(9H,s,$^+N(CH_3)_3$)3.76-4.03(2H,m, $CH_2N^+$)4.99(2H,d,$CH_2$-CH=)5.75-5.80(1H,m,CHOR)5.92 (1H,t,$CH_2$-CH=)7.53-7.80(3H,m,Arom) | $C_{21}H_{27}BrFNO_7$<br>C H Br N<br>calc% 51.65 5.57 16.36 2.87<br>fou.% 51.53 5.49 16.14 2.69 |
| ST 900 Ex.20 | 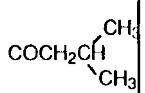 | F | 154-155°C | -9,6 | 86 | 3.45 | 0.88(9H,t,$CH(CH_3)_2$)1.95-2.04(1H,m,$CH(CH_3)_2$2.32 (2H,d,$CH_2CH(CH_3)_2$)2.95-3.13(2H,m,$CH_2COOR$)3.32 (9H,s,$^+N(CH_3)_3$)3.89-4.14(2H,m,$CH_2N^+$)5.01(2H,d,$CH_2$-CH=) 5.82-5.88(1H,m,CHOR)5.93-5.98(1H,t,$CH_2$-CH=)7.46-7.87 (3H,m,arom) | $C_{22}H_{29}BrFNO_6$<br>C H Br N<br>calc% 52.60 5.82 15.91 2.79<br>fou.% 52.66 5.82 15.94 2.87 |

TABLE 1 (continuation)

| Comp. | R | $R_2$ | M.P. °C | $\lvert\alpha\rvert^{25}_{D}$ (C = 1 %$H_2O$) | yield % | HPLC* K' | $^1$H-NMR Varian 300 MHz ($D_2O$) δ (p.p.m.) | ELEMENTARY ANALYSIS |
|---|---|---|---|---|---|---|---|---|
| ST 942 Ex.21 | $COCH_3$ | $NO_2$ | 134°C (dec.) | - 10.0 | 52 | 3.34 | 2.16(3H,s,$COC\underline{H}_3$)2.99(2H,d,$C\underline{H}_2COOR$)3.26 (9H,s,$^+N(C\underline{H}_3)_3$)3.77-3.99(2H,m,$C\underline{H}_2N+$)5.06 (2H,d,$C\underline{H}_2$-CH=)5.74(1H,m,$C\underline{H}OR$)6.21(1H,t,$CH_2$-$C\underline{H}$=) 8.01(1H,d,Arom)8.58(1H,dd,Arom)8.66(1H,d,Arom) | $C_{19}H_{23}BrN_2O_8$<br>C H Br N<br>calc% 46.83 4.76 16.40 5.75<br>fou.% 46.88 4.84 16.42 5.69 |
| ST 934 Ex.22 | $COCH_2\underset{CH_3}{\overset{CH_3}{CH}}$ | $NO_2$ | 139-140°C | - 6.0 | 65 | 14.19 | 0.85(6H,t,$CH(C\underline{H}_3)_2$)1.98(1H,m,$C\underline{H}(CH_3)_2$)2.32 (2H,d,$C\underline{H}_2CH(CH_3)_2$)3.04(2H,d,$C\underline{H}_2COOR$)3.37 (9H,s,$^+N(C\underline{H}_3)_3$)3.92-4.09(2H,m,$C\underline{H}_2N+$)5.03 (2H,d,$C\underline{H}_2CH$=)5.83(1H,m,$C\underline{H}OR$)6.27(1H,t,$CH_2$-$C\underline{H}$=) 8.10(1H,d,Arom)8.47(1H,s,Arom)8.56(1H,d,Arom) | $C_{22}H_{29}BrN_2O_8$<br>C H Br N<br>calc% 49.91 5.52 15.09 5.29<br>fou. % 49.81 5.59 14.91 5.23 |

TABLE 1 (continuation)

| Comp. | R | $R_3$ | M.P. °C | $\|\alpha\|^{25}_{D}$ (C = 1 %$H_2O$) | yield % | HPLC' K' | $^1$H-NMR Varian 300 MHz ($D_2O$) δ (p.p.m.) | ELEMENTARY ANALYSIS |
|---|---|---|---|---|---|---|---|---|
| ST 901 Ex.23 | H | Cl | 201-204°C | - 11.0 | 80 | 2.46 | 2.81 (2 H, m,$CH_2COOR$)3.29 (9H,s,$^+N(CH_3)_3$) 3.58(2H,m,$CH_2N+$)4.8(1H,m,CHOH)5.01(2H,d,$CH_2$-CH=) 5.89(1H,t,$CH_2$-CH=)7.54-7.66(3H,m,Arom) | $C_{17}H_{21}BrClNO_5$<br>C H Br Cl N<br>calc% 46.97 4.87 18.38 8.16 3.22<br>fou.% 46.81 5.03 18.48 8.02 3.12 |
| ST 905 Ex.24 | $COCH_3$ | Cl | 102-105°C | - 10.0 | 90 | 4.95 | 2.15(3H,s,$COCH_3$)2.97(2H,m,$CH_2COOR$)3.25(9H,s,$^+N(CH_3)_3$) 3.75-3.98(2H,m,$CH_2N+$)5.00(2H,d,$CH_2$-CH=)5.73 (1H,m,CHOR) 5.90(1H,t,$CH_2$-CH=)7.57-7.72(3H,m,Arom) | $C_{19}H_{23}BrClNO_6$<br>C H Br Cl N<br>calc% 47.87 4.86 16.76 7.44 2.94<br>fou. % 47.81 4.95 16.63 7.41 2.99 |
| ST 903 Ex.25 | $COCH_2CH_3$ | Cl | 176-178°C | - 10.6 | 76 | 7.99 | 1.07(3H,t,$COCH_2CH_3$)2.44(2H,m,$COCH_2CH_3$)2.97 (2H,m,$CH_2COOR$)3.26 (9H,s,$^+N(CH_3)_3$)3.78-3.99 (2H,m,$CH_2N+$)4.96(2H,d,$CH_2$-CH=)5.76(1H,m,CHOR) 5.86(1H,t,$CH_2$-CH=)7.52-7.65(3H,m,Arom) | $C_{20}H_{25}BrClNO_6$<br>C H Br Cl N<br>calc% 48.95 5.13 16.28 7.22 2.85<br>fou. % 49.91 5.15 16.09 7.20 2.95 |
| ST 902 Ex.26 | $CH_3$<br>COCH<br>$CH_3$ | Cl | 194-196°C | -8.8 | 87 | 12.59 | 1.12(6H,d,CH($CH_3$)$_3$2.65(1H,m,CH($CH_3$)$_2$)2.98 (2H,m,$CH_2COOR$)3.27 (9H,s,$^+N(CH_3)_3$)3.81-4.03 (2H,m,$CH_2N+$)4.98(2H,d,$CH_2$-CH=)5.79(1H,m,CHOR) 5.89 (1H,t,$CH_2$-CH=)7.54-7.70(3H,m,Arom) | $C_{21}H_{27}BrClNO_6$<br>C H Br Cl N<br>calc% 49.97 5.39 15.83 7.02 2.77<br>fou.% 49.89 5.51 15.81 6.93 2.85 |
| ST 899 Ex.27 | $CH_3$<br>$COCH_2CH$<br>$CH_3$ | Cl | 188-189°C | - 7.1 | 82 | 22.04 | 0.87(6H,t,CH($CH_3$)$_2$1.99(1H,m,CH($CH_3$)$_2$)2.31 (2H,d,$CH_2$-CH($CH_3$)$_2$)3.04(2H,m,$CH_2COOR$)3.37 (9H,s,$^+N(CH_3)_3$)4.01(2H,m,$CH_2N+$)4.99(2H,d,$CH_2$-CH=) 5.84(1H,m,CHOR) 5.92(1H,t,$CH_2$-CH=)7.45-7.68 (3H,m,Arom) | $C_{22}H_{29}BrClNO_6$<br>C H Br Cl N<br>calc% 50.93 5.63 15.40 6.83 2.70<br>fou.% 51.00 5.71 15.44 6.75 2.71 |

TABLE 1 (continuation)

| Comp. | R | $R_2$ | M.P. °C | $[\alpha]^{25}_{D}$ (C = 1 % $H_2O$) | yield % | HPLC* K' | $^1H$-NMR Varian 300 MHz ($D_2O$) δ (p.p.m.) | ELEMENTARY ANALYSIS |
|---|---|---|---|---|---|---|---|---|
| ST 876 Ex.28 | H | Cl | 148-153°C | +8.5 | 70 | 2.87 | 2.85 (2 H, m, $CH_2COOR$) 3.33 (9H, s, $^+N(CH_3)_3$) 3.62 (2H, m, $CH_2N^+$) 4.83 (1H, m, CHOH) 5.11 (2H, d, $CH_2$-CH=) 6.04 (1H, t, $CH_2$-CH=) 7.77-7.85 (3H, m, Arom) | $C_{17}H_{21}BrClNO_5$<br>C H Br Cl N<br>calc% 46.97 4.87 18.38 8.16 3.22<br>fou. % 46.81 4.91 18.35 8.02 3.17 |
| ST 875 Ex.29 | H | H | 152-156°C (dec.) | +9.5 | 75 | 1.16 | 2.81 (2H, m, $CH_2COOR$) 3.32 (9H, s, $^+N(CH_3)_3$) 3.59 (2 H, m, $CH_2N^+$) 4.80 (1H, m, CHOH) 4.97 (2H, dd, $CH_2$-CH=) 5.84 (1H, t, $CH_2$-CH=) 7.57-7.6.7 (3H, m, Arom) | $C_{17}H_{22}BrNO_5$<br>C H Br N<br>calc% 51.01 5.54 19.96 3.50<br>fou. % 50.99 5.64 19.79 3.46 |

ST 876, 875: have D configuration

TABLE 1 (continuation)

| Comp. | R | Y | $R_2$ | M.P. °C | $[\alpha]^{25}_{D}$ (C = 1 %$H_2O$) | yield% | HPLC* K' | $^1H$-NMR Varian 300 MHz ($D_2O$) δ (p.p.m.) | ELEMENTARY ANALYSIS |
|---|---|---|---|---|---|---|---|---|---|
| ST 922 Ex.30 | H | $(CH_2)_2$ | Cl | 193°C (dec.) | - 10.4 | 73 | 2.05 | 2.70 (2 H, m,$CH_2C\underline{H}_2$-CH=)2.77(2 H,m,$C\underline{H}_2$COOR) 3.25 (9H,s,$^+N(C\underline{H}_3)_3$)3.51(2H,m,$C\underline{H}_2N^+$)4.36(2H,t,$CH_2C\underline{H}_2O$)4.70 (1H,m,$C\underline{H}$OH)5.83(1H,t,$CH_2$-$C\underline{H}$=)7.60(3H,m,Arom) | $C_{18}H_{23}Cl_2NO_5$<br>C H Cl N<br>calc% 53.48 5.73 17.54 3.46<br>fou.% 53.39 5.80 17.56 3.40 |
| ST 911 Ex.31 | 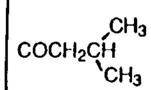 | $(CH_2)_2$ | Cl | 69°C (dec.) | -9.6 | 84 | 14.35 | 0.80(6H,d,$CH(C\underline{H}_3)_2$)1.89(1H,m,$C\underline{H}(CH_3)_2$)2.18(2H,d, $C\underline{H}_2CH(CH_3)_2$)2.73(2H,m,$CH_2C\underline{H}_2$-CH=)2.85(2 H,m,$C\underline{H}_2$COOR) 3.22(9H,s,$^+N(C\underline{H}_3)_3$3.76-3.92(2H,m,$C\underline{H}_2N^+$) 4.28(2H,m,$CH_2C\underline{H}_2O$)5.66(1H,m,$C\underline{H}$OR)5.80(1H,t,$CH_2$-$C\underline{H}$=) 7.57(3H,m,Arom) | $C_{23}H_{31}Cl_2NO_6$<br>C H Br N<br>calc% 56.56 6.40 14.52 2.87<br>fou.% 56.42 6.49 14.41 2.83 |

ST 922, 911: $X^- = Cl^-$

K' = capacity factor

* ST 739, 775, 776, 866, 871, 869, 870, 867, 901, 905, 903, 902, 899, 942, 934, 876, 875, 933, 917: Waters 990-column μ Bondapak $C_{18}$, inner diameter = 3.9 mm; length = 300 mm; mobile phase = $KH_2PO_4$ 0.05 M/$CH_3CN$ 70:30; t = 25°C; flow rate = 1ml/min; detector: U.V.λ = 205 nm; capacity factor calculated on the peak of $Br^-$ anion.

* ST 900: same conditions with mobile phase: $KH_2PO_4$0.05 M/$CH_3CN$ 60:40

* ST 922, 911: same conditions with mobile phase: $KH_2PO_4$ 0.05 M/$CH_3CN$ 65:35, capacity factor calculated on the peak of $Cl^-$ anion.

* ST 768: same conditions with flow rate 1.5 ml/min; capacity factor calculated on the peak of $Br^-$ anion.

* ST 801, 804, 805, 806, 803: Waters 990-column μ Bondapak $C_{18}$, inner diameter = 4 mm, length = 300 mm; mobile phase = $KH_2PO_4$ 0.05 M/$CH_3CN$ 65:35; t = 25°C; flow rate = 1.5 ml/min; detector: U.V.λ= 205 nm; capacity factor calculated on the peak of $Br^-$ anion.

* ST 931: Waters 481 - column Spherisorb - $C_1$ (5μ) inner diameter 4.6 mm, length = 250 mm; mobile phase = $CH_3CN$/$KH_2PO_4$ 0.01M 60:40, pH = 4 with $H_3PO_4$; t = 30°C; flow rate = 1 ml/min; detector: U.V.λ= 205 nm; capacity factor calculated on the peak of $Br^-$ anion.

* ST 926: Waters 481 - column Spherisorb - SCX (5μ); inner

**diameter 4 mm, length = 250 mm; mobile phase = $CH_3CN/KH_2PO_4$ 0.05 M 65:35, pH = 4.2 with $H_3PO_4$; t= 40°C; flow rate 1 ml/min; detector: U.V. $\lambda$ = 205 nm; capacity factor calculated on the peak of $Br^-$ anion.**

In synthesizing the compounds of the invention, the selection of solvents and operating conditions can be broadened with respect to those set forth in Example 1, as hereinbelow disclosed.

As reaction solvents, aprotic dipolar solvents are used such as e.g. dimethylformamide, acetonitrile, diethyleneglycol dimethyl ether, dimethyl sulfoxide, sulfolane, preferably dimethylformamide.

The reaction temperature is comprised between +5°C and +80°C, preferably between +25°C and +35°C.

The dropping time is comprised between 10 and 120 minutes and preferebly is 60 minutes.

The reaction time is comprised between 1 and 48 hours, preferably 2-3 hours.

When the reaction is completed, the stirring temperature is comprised between -20°C and +25°C, preferably +5°C, for times ranging from 2 to 48 hours, preferably 14 hours.

The solvents for precipitating and isolating the final compounds are selected from symmetrical or asimmetrical, straight or branched lower alkyl ethers, such as diethyl ether, di -n-propyl ether, diisopropyl ether, di-n-butyl ether, preferably diethyl ether.

Alternatively, the reaction mixture can be concentrated under vacuum at low temperature, and the residue recrystallized from suitable solvent(s).

Some of the pharmacological tests conducted in order to assess the activity of the compounds of the present invention are reported hereinbelow.

BINDING TO PAF RECEPTORS

The binding of the compounds under examination to rabbit platelet receptors was studied following the procedure disclosed by D. Nunez et al, Eur J. Pharmacol. 123, 197 (1986).

Blood was drawn via intracardiac puncture from male rabbits (HY/CR strain-Charles River, Italy) weighing 2.5-3.0 Kg.

Blood was diluted (1:9) in a ACD (0.8% citric acid, 2.2% trisodium citrate, 2.45% glucose, weight percentages) solution and centrifuged at 100 x g for 15 minutes.

The precipitate was suspended in the incubation buffer at $2^{-4} \times 10^8$ platelet/ml concentration, immediately before binding.

The platelet number and possible erythrocyte contamination was controlled with a platelet counter DELCON CA480.

Platelets were incubated in polyethylene tubes with $^3$H-PAF (0.5 nM) for 30 minutes at 25°C. The test compounds were added to a volume of 200 µl. The final volume was equal to 500 µl. Incubation was stopped by rapid filtration under vacuum through GF/C filters (WHATMAN) washed with (3x3 ml) cold incubation buffer using a Brondel Cell Harvester filtration system. The filters were counted in 8 mL OptiFluor (Packard) using a TRI-CARB 1900 CA-Packard liquid scintillation spectrometer (about 50% counting efficiency).

RESULTS EVALUATION

The percentage of inhibition of the specific binding receptor-radioligand was calculated for each concentration (mean of incubations in triplicate).

The competition curve, the slope of the curve, and the $IC_{50}$ value (concentration which inhibits 50% of the specific radioligand-receptor binding) were calculated with the "All fit" program (A. De Lean et al., Am. J. Physiol. 235, E97, 1978) runned in a 55-SX IBM computer. The results are shown in Table 2.

PLATELET AGGREGATION ANTAGONISM INDUCED BY PAF (Platelet activating factor) and ADP (Adenosinediphosphate)

PAF-INDUCED AGGREGATION

Blood was collected from male rabbits (HY/Charles River, Italy) by heart puncture using a plastic syringe and transferred immediately into plastic tubes containing 3.8% (by weight) solution of sodium citrate (0.1 ml/ml blood).

PRP (Platelet Rich Plasma) was obtained by plasma centrifugation at 145 x g for 5 minutes at 22°C. Platelet number was determined with a CA 580A (Delcon) "platelet counter". The platelet number was adjusted to the fixed value of 350,000 platelet/ml by adding PPP (Platelet Poor Plasma). PPP was obtained by centrifuging blood at 1600 x g for 10 minutes at 22°C.

Platelet aggregation was determined photometrically (G.V.R. Born, Nature 194, 927; 1962) with an ELVI 840 aggregometer in plastic tubes under continuous stirring at 1000 rpm.

Maximal light transmission (100%) was recorded by reading PPP, while 0% transmission was recorded by reading PRP. The PRP aggregation degree induced by the aggregation factor (PAF) was calculated as percentage of maximal light transmittance obtained with PPP using 250 $\mu$L samples of PRP.

For each test, the concentration of aggregating agent needed to obtain 50-60% aggregation was determined and was shown to range between $10^{-7}$ and 9 x $10^{-8}$ M.

The compounds were dissolved in bidistilled water at the concentration of $10^{-5}$ M and tested after 2 minutes of incubation.

Aggregation inhibition was calculated as percentage of the value obtained in the presence of the aggregating agent only.

ADP-INDUCED AGGREGATION

Blood was drawn from the abdominal aorta of male rats (Crl:(WI)BR, Charles River, Italy) under barbiturate anaesthesia and quickly transferred into plastic tubes containing 0.1 mL/mL blood of 3.8% (by weight) solution of sodium citrate. PRP was obtained by plasma centrifugation at 115 x g for 5 minutes at 22°C and PPP by centrifugation at 1600 x g for 10 minutes. The final PRP platelet number was adjusted to the fixed value of 350,000/mL of PRP by adding PPP.

Platelet count was made with a CA580 A(Delcan) platelet counter. The same procedure outlined in the paragraph "PAF aggregation" was followed for the ADP-induced aggregation. The concentration of the aggregating factor (ADP) ranged from 5 x $10^{-6}$ to 1 x $10^{-5}$ M. The results are shown in Table 4.

OBSERVATION OF THE BEHAVIOUR AND DEATH RATE IN MICE

The observation of normal behaviour in mice was conducted following the method by S. Irwin, Psychopharmacologia 13, 222 ( 1968). This method allows alterations of some behavioural, neurophysiological and neurovegetative parameters directly detectable by the experimenter to be visualized.

The test was conducted in male mice [Crl;(CD-I)(ICR)BR, Charles River - Italy] weighing 22-25 g, following oral administration of the compounds suspended in carboxymethylcellulosa (0.5% by weight in $H_2O$) to groups of 4 animals/dose.

The animals were kept under continuous observation for five hours following treatment and twice a day for the subsequent five days.

Death rate was also observed during the whole test period. The initial administered dose was 1,000 mg/kg per os; lower doses were administered following death of some animals or after marked effects at the initial dose were dected.

The results are shown in Table 3.

CONCLUSION

The compounds were shown to be active in antagonizing PAF binding to its receptors at pharmacologically meaningful concentrations. The most active ones among the compounds were subsequently tested in the PAF-induced platelet aggregation test in order to assess whether the compounds behave as agonists or antagonists towards PAF receptors. The results obtained showed that the compounds are antagonists. Moreover, it was shown that their activity is specific because the same compounds were inactive in antagonizing the aggregation triggered by an aggregating agent (such as ADP) endowed with a different

mode of action.

Table 2 Binding to PAF (platelet activating factor) receptors in rabbit platelets

General Formula 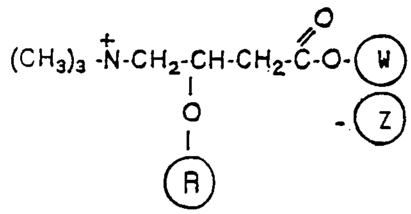 or

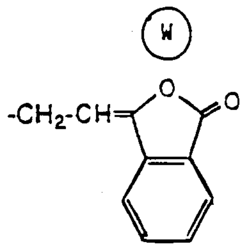

| 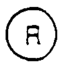 | | $IC_{50}$ (*) ± d.s. [M] |
|---|---|---|
| -H | ST 736 | $9.3 \times 10^{-6}$ ± 1.3 |
| $-CO-CH_3$ | ST 739 | $5.3 \times 10^{-6}$ ± 0.8 |
| $-CO-CH_2-CH_3$ | ST 768 | $6.7 \times 10^{-6}$ ± 1.7 |
| 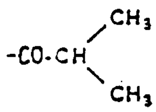 | ST 775 | $2.0 \times 10^{-6}$ ± 0.5 |
| 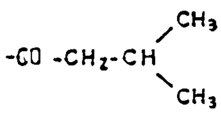 | ST 776 | $3.6 \times 10^{-6}$ ± 1.0 |

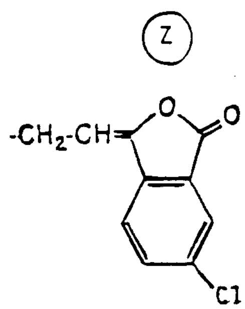

| 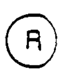 | | |
|---|---|---|
| -H | ST 801 | $2.0 \times 10^{-8}$ ± 0.7 |
| $-CO-CH_3$ | ST 804 | $1.9 \times 10^{-8}$ ± 0.6 |
| $-CO-CH_2-CH_3$ | ST 805 | $2.2 \times 10^{-8}$ ± 0.7 |
| 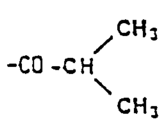 | ST 806 | $1.8 \times 10^{-8}$ ± 0.5 |
| 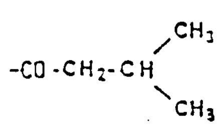 | ST 803 | $6.5 \times 10^{-9}$ ± 2.4 |

*$IC_{50}$ = 50% inhibiting concentration of specific receptor binding

Table 3

| Observation of behaviour and death rate in mice | | | |
|---|---|---|---|
| COMPOUND | DOSE mg/kg per os | SYMPTOMS | DEATH RATE |
| ST 736 | 600<br>1000 | n.e.<br>convulsion, salivation | 0/4<br>3/4 |
| ST 739 | 600<br>1000 | n.e.<br>jerks | 0/4<br>2/4 |
| ST 768 | 600<br>1000 | n.e.<br>convulsion, salivation diarrhea, lacrimation | 0/4<br>1/4 |
| ST 775 | 1000 | n.e. | 0/4 |
| ST 776 | 600<br>1000 | n.e.<br>convulsion, salivation | 0/4<br>2/4 |
| ST 801 | 1000 | salivation | 0/4 |
| ST 804 | 600<br>1000 | salivation<br>salivation | 0/4<br>1/4 |
| ST 805 | 600<br>1000 | salivation<br>salivation | 0/4<br>1/4 |
| ST 806 | 600<br>1000 | salivation<br>salivation | 0/4<br>1/4 |
| ST 803 | 1000 | diarrhea | 0/4 |
| n.e. = no effect | | | |

Table 4

| Inhibition to PAF(°)- and ADP(°°)-induced platelet aggregation | | | |
|---|---|---|---|
| COMPOUND | CONCENTRATION | % INHIBITION | |
| | | PAF | ADP |
| ST 776 | $10^{-5}$ M | 100% | 5% |
| ST 801 | " | 97% | 7% |
| ST 804 | " | 96% | 13% |
| ST 805 | " | 40% | 10% |
| ST 806 | " | 95% | 18% |
| ST 803 | " | 72% | 8% |

(°) PAF = platelet activating factor
(°°) ADP = adenosinediphosphate

## Claims

**Claims for the following Contracting States : AT, BE, CH, LI, DE, DK, FR, GB, IT, LU, NL, SE**

1. (3-phthalidylidene) alkyl esters of carnitine and alkanoyl carnitines of general formula (I)

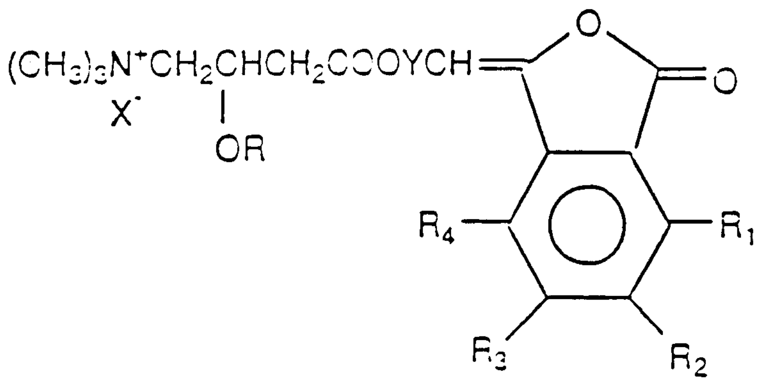

(I)

wherein:

Y is a $C_1$-$C_5$ alkylene group, unsubstituted or substituted with one or more $C_1$-$C_4$ lower alkyl groups;

R is hydrogen or $C_2$-$C_6$ alkanoyl;

$R_1$, $R_2$, $R_3$ and $R_4$, identical or different, are hydrogen, halogen, $C_1$-$C_8$ alkoxy, lower $C_1$-$C_4$ alkyl, halogen-substituted lower alkyl amino, alkyl-substituted amino wherein the alkyl group has 1 to 4 carbon atoms, nitro, cyano, $C_1$-$C_4$ alkanoylamino, or $R_1$ and $R_2$ taken together, $R_2$ and $R_3$ taken together or $R_3$ and $R_4$ taken together form a $C_1$-$C_4$ alkylenedioxy group, and

$X^-$ is the anion of a pharmacologically acceptable salt.

2. The esters of claim 1, wherein Y is methylene or ethylene.

3. The esters of claim 1 or 2, wherein R is selected from acetyl, propionyl, butyryl, isobutyryl, valeryl and isovaleryl.

4. The esters of anyone of the preceding claims, wherein the halogen is selected from fluorine and chlorine.

5. The esters of anyone of the preceding claims, wherein the alkoxy group is selected from methoxy and ethoxy.

6. The esters of anyone of the preceding claims, wherein the halogen-substituted lower alkyl is selected from trifluoromethyl and trichloromethyl.

7. The esters of anyone of the preceding claims, wherein the alkanoylamino is acetylamino.

8. The esters of claim 1, wherein Y is methylene, $R_1 = R_2 = R_3 = R_4 =$ hydrogen and R is selected from hydrogen, acetyl, propionyl, butyryl, isobutyryl, valeryl and isovaleryl.

9. The esters of claim 1, wherein Y is methylene, any three groups from $R_1$, $R_2$, $R_3$ and $R_4$ are hydrogen and the remaining group is fluorine or chlorine, and R is selected from hydrogen, acetyl, propionyl, butyryl, isobutyryl, valeryl and isovaleryl.

10. The esters of claim 9, wherein $R_1 = R_3 = R_4 =$ hydrogen and $R_2$ is fluorine or bromine.

11. A pharmaceutical composition comprising as active ingredient a compound according to anyone of the preceding claims, and a pharmacologically acceptable excipient therefor.

**12.** The pharmaceutical composition of claim 11 for the treatment of diseases whereof PAF is the etiological agent or is one of the etiological agents.

**13.** The pharmaceutical composition of claim 12 for the treatment of inflammatory and allergic conditions.

**14.** The pharmaceutical composition of claim 13 for the treatment of asthma.

**15.** The pharmaceutical composition of claim 12 for the treatment of shock.

**16.** The pharmaceutical composition of claim 15 for the treatment of anaphylactic shock.

**17.** The pharmaceutical composition of claim 11 as contraceptive.

**18.** The pharmaceutical composition of claim 11 for the treatment of pre-senile and senile dementias.

**19.** The pharmaceutical composition of claim 18 for the treatment of Alzheimer's disease.

**Claim for the following Contracting States : ES, GR**

**1.** A process for producing (3-phthalidylidene) alkyl esters of carnitine and alkanoyl carnitines of general formula (I)

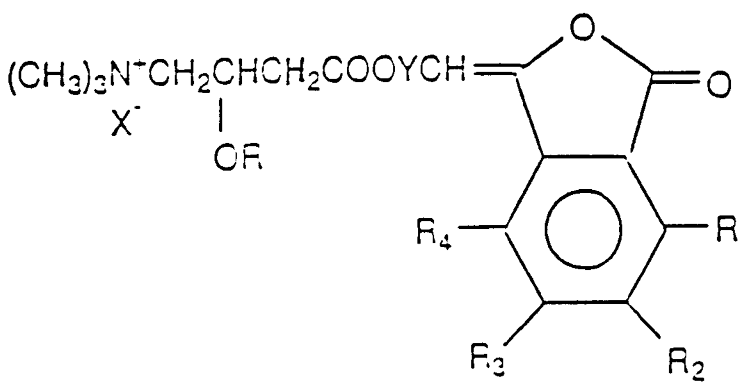

(I)

wherein:

| | |
|---|---|
| Y | is a $C_1$-$C_5$ alkylene group, unsubstituted or substituted with one or more lower $C_1$-$C_4$ alkyl groups; |
| R | is hydrogen or $C_2$-$C_6$ alkanoyl; |
| $R_1$, $R_2$,$R_3$ and $R_4$, | identical or different, are hydrogen, halogen, $C_1$-$C_8$ alkoxy, $C_1$-$C_4$ lower alkyl, halogen-substituted lower alkyl, amino, alkyl-substituted amino wherein the alkyl group has 1 to 4 carbon atoms, nitro, cyano, $C_1$-$C_4$ alkanoylamino, or $R_1$ and $R_2$ taken together, $R_2$ and $R_3$ taken together or $R_3$ and $R_4$ taken together form a $C_1$-$C_4$ alkylenedioxy group, and $X^-$ is the anion of a pharmacologically acceptable salt, which comprises condensing carnitine or an alkanoylcarnitine wherein the alkanoyl group has 2 to 6 carbon atoms with 3-($\omega$-bromoalkylidene) phtalide which is optionally substituted at 4,5,6 and 7 positions thereof with, respectively, the previously defined $R_4$,$R_3$,$R_2$ and $R_1$ groups, in the presence of aprotic, dipolar solvents, at a temperature from +5 to +80 °C, for reaction times from 1 to 48 hours. |

**Patentansprüche**

**Patentansprüche für folgende Vertragsstaaten : AT, BE, CH, LI, DE, DK, FR, GB, IT, LU, NL, SE**

1. (3-Phthalidyliden)alkylester von Carnitin und Alkanoylcarnitinen der allgemeinen Formel (I)

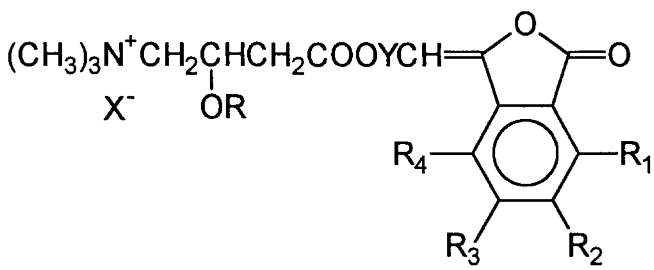

(I)

worin:
Y eine $C_{1-5}$-Alkylengruppe ist, die unsubstituiert oder mit einer oder mehreren $C_{1-4}$-Niederalkylgruppen substituiert ist;
R Wasserstoff oder $C_{2-6}$-Alkanoyl ist;
$R_1$, $R_2$, $R_3$ und $R_4$ gleich oder verschieden Wasserstoff, Halogen, $C_{1-8}$-Alkoxy, $C_{1-4}$-Niederalkyl, halogensubstituiertes Niederalkyl, Amino, alkylsubstituiertes Amino, wobei die Alkylgruppe 1 bis 4 Kohlenstoffatome hat, Nitro, Cyano, $C_{1-4}$-Alkanoylamino sind, oder $R_1$ und $R_2$ zusammen, $R_2$ und $R_3$ zusammen oder $R_3$ und $R_4$ zusammen eine $C_{1-4}$-Alkylendioxygruppe bilden; und
$X^-$ das Anion eines pharmakologisch annehmbaren Salzes ist.

2. Ester gemäss Anspruch 1, bei denen Y Methylen oder Ethylen ist.

3. Ester nach Anspruch 1 oder 2, wobei R ausgewählt ist aus Acetyl, Propionyl, Butyryl, Isobutyryl, Valeryl und Isovaleryl.

4. Ester nach einem der vorhergehenden Ansprüche, bei denen das Halogen ausgewählt ist aus Fluor und Chlor.

5. Ester nach einem der vorhergehenden Ansprüche, in denen die Alkoxygruppe ausgewählt ist aus Methoxy und Ethoxy.

6. Ester nach einem der vorhergehenden Ansprüche, in denen die halogensubstituierte Niederalkylgruppe ausgewählt ist aus Trifluormethyl und Trichlormethyl.

7. Ester nach einem der vorhergehenden Ansprüche, in denen die Alkanoylaminogruppe Acetylamino ist.

8. Ester gemäss Anspruch 1, in denen Y Methylen ist, $R_1 = R_2 = R_3 = R_4$ = Wasserstoff ist, und R ausgewählt ist aus Wasserstoff, Acetyl, Propionyl, Butyryl, Isobutyryl, Valeryl und Isovaleryl.

9. Ester gemäss Anspruch 1, in denen Y Methyl ist, beliebige drei Gruppen aus $R_1$, $R_2$, $R_3$ und $R_4$ Wasserstoff sind und die übrige Gruppe Fluor oder Chlor ist, und R ausgewählt ist aus Wasserstoff, Acetyl, Propionyl, Butyryl, Isobutyryl, Valeryl und Isovaleryl.

10. Ester nach Anspruch 9, in denen $R_1 = R_3 = R_4$ = Wasserstoff und $R_2$ Fluor oder Brom ist.

11. Pharmazeutische Zusammensetzung, umfassend als Wirkstoff eine Verbindung gemäss einem der vorhergehenden Ansprüche und einen dafür pharmakologisch annehmbaren Träger.

12. Pharmazeutische Zusammensetzung nach Anspruch 11 zur Behandlung von Krankheiten, bei denen PAF das ätiologische Agens oder eines der ätiologischen Agenzien ist.

**13.** Pharmazeutische Zusammensetzung nach Anspruch 12 zur Behandlung von Entzündungs- und allergischen Leiden.

**14.** Pharmazeutische Zusammensetzung nach Anspruch 13 zur Behandlung von Asthma.

**15.** Pharmazeutische Zusammensetzung nach Anspruch 12 zur Behandlung von Schock.

**16.** Pharmazeutische Zusammensetzung nach Anspruch 15 zur Behandlung von anaphylaktischem Schock.

**17.** Pharmazeutische Zusammensetzung nach Anspruch 11 als Kontrazeptivum.

**18.** Pharmazeutische Zusammensetzung nach Anspruch 11 zur Behandlung von präsenilen und senilen Demenzen.

**19.** Pharmazeutische Zusammensetzung nach Anspruch 18 zur Behandlung der Alzheimer'schen Krankheit.

**Patentanspruch für folgende Vertragsstaaten : ES, GR**

**1.** Verfahren zur Herstellung von (3-Phthalidyliden)alkylestern von Carnitin und Alkanoylcarnitinen der allgemeinen Formel (I)

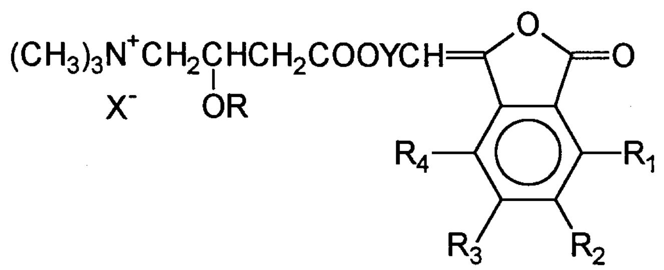

(I)

worin:
Y eine $C_{1-5}$-Alkylengruppe ist, die unsubstituiert oder mit einer oder mehreren $C_{1-4}$-Niederalkylgruppen substituiert ist;
R Wasserstoff oder $C_{2-6}$-Alkanoyl ist;
$R_1$, $R_2$, $R_3$ und $R_4$ gleich oder verschieden Wasserstoff, Halogen, $C_{1-8}$-Alkoxy, $C_{1-4}$-Niederalkyl, halogensubstituiertes Niederalkyl, Amino, alkylsubstituiertes Amino, wobei die Alkylgruppe 1 bis 4 Kohlenstoffatome hat, Nitro, Cyano, $C_{1-4}$-Alkanoylamino sind, oder $R_1$ und $R_2$ zusammen, $R_2$ und $R_3$ zusammen oder $R_3$ und $R_4$ zusammen eine $C_{1-4}$-Alkylendioxygruppe bilden; und
$X^-$ das Anion eines pharmakologisch annehmbaren Salzes ist, umfassend die Kondensation von Carnitin oder eines Alkanoylcarnitins, in dem die Alkanoylgruppe 2 bis 6 Kohlenstoffatome hat, mit 3-(ω-Bromalkyliden)phthalid, das gegebenenfalls an den 4-, 5-, 6- und 7-Positionen mit den zuvor definierten $R_4$-, $R_3$-, $R_2$- bzw. $R_1$-Gruppen substituiert ist, in Gegenwart von aprotischen dipolaren Lösungsmitteln bei einer Temperatur von +5 bis +80°C während Reaktionszeiten von 1 bis 48 Stunden.

## Revendications

**Revendications pour les Etats contractants suivants : AT, BE, CH, LI DE, DK, FR, GB, IT, LU, NL, SE**

**1.** Esters (3-phtalidylidène)alkyliques de carnitine et d'alcanoylcarnitines de formule générale (I)

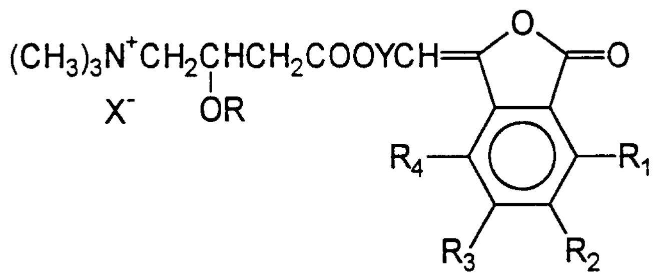

(I)

dans laquelle :

| | |
|---|---|
| Y | est un groupe alkylène en $C_1$-$C_5$ non substitué ou substitué avec un ou plusieurs groupes alkyle inférieurs en $C_1$-$C_4$ ; |
| R | est un atome d'hydrogène ou un groupe alcanoyle en $C_2$-$C_6$ ; |
| $R_1$, $R_2$, $R_3$ et $R_4$ | qui sont identiques ou différents, sont un atome d'hydrogène ou d'halogène ou un groupe alcoxy en $C_1$-$C_8$, alkyle inférieur en $C_1$-$C_4$, alkyle inférieur halogéno-substitué, amino, amino alkyle-substitué dont le groupe alkyle a 1 à 4 atomes de carbone, nitro, cyano ou alcanoylamino en $C_1$-$C_4$, ou $R_1$ et $R_2$ pris ensemble, $R_2$ et $R_3$ pris ensemble ou $R_3$ et $R_4$ pris ensemble forment un groupe alkylènedioxy en $C_1$-$C_4$, et |
| $X^-$ | est l'anion d'un sel pharmacologiquement acceptable. |

**2.** Esters selon la revendication 1, où Y est un groupe méthylène ou éthylène.

**3.** Esters selon la revendication 1 ou 2, où R est choisi parmi les groupes acétyle, propionyle, butyryle, isobutyryle, valéryle et isovaléryle.

**4.** Esters selon l'une quelconque des revendications précédentes, où l'halogène est choisi parmi le fluor et le chlore.

**5.** Esters selon l'une quelconque des revendications précédentes, où le groupe alcoxy est choisi parmi les groupes méthoxy et éthoxy.

**6.** Esters selon l'une quelconque des revendications précédentes, où le groupe alkyle inférieur halogéno-substitué est choisi parmi les groupes trifluorométhyle et trichlorométhyle.

**7.** Esters selon l'une quelconque des revendications précédentes, où le groupe alcanoylamino est le groupe acétylamino.

**8.** Esters selon la revendication 1, où Y est un groupe méthylène, $R_1$ = $R_2$ = $R_3$ = $R_4$ = un atome d'hydrogène et R est choisi parmi un atome d'hydrogène et un groupe acétyle, propionyle, butyryle, isobutyryle, valéryle et isovaléryle.

**9.** Esters selon la revendication 1, où Y est un groupe méthylène, trois quelconques des symboles $R_1$, $R_2$, $R_3$ et $R_4$ sont des atomes d'hydrogène et le symbole restant est un atome de fluor ou de chlore, et R est choisi parmi un atome d'hydrogène et un groupe acétyle, propionyle, butyryle, isobutyryle, valéryle et isovaléryle.

**10.** Esters selon la revendication 9, où $R_1$ = $R_3$ = $R_4$ = un atome d'hydrogène et $R_2$ est un atome de fluor ou de brome.

**11.** Composition pharmaceutique comprenant, comme ingrédient actif, un composé selon l'une quelconque des revendications précédentes et un excipient pharmacologiquement acceptable de celui-ci.

**12.** Composition pharmaceutique selon la revendication 11 pour le traitement des maladies dont le PAF est l'agent étiologique ou un des agents étiologiques.

**13.** Composition pharmaceutique selon la revendication 12 pour le traitement des états inflammatoires et allergiques.

**14.** Composition pharmaceutique selon la revendication 13 pour le traitement de l'asthme.

**15.** Composition pharmaceutique selon la revendication 12 pour le traitement du choc.

**16.** Composition pharmaceutique selon la revendication 15 pour le traitement du choc anaphylactique.

**17.** Composition pharmaceutique selon la revendication 11 en tant que contraceptif.

**18.** Composition pharmaceutique selon la revendication 11 pour le traitement des démences préséniles et séniles.

**19.** Composition pharmaceutique selon la revendication 18 pour le traitement de la maladie d'Alzheimer.

**Revendication pour les Etats contractants suivants : ES, GR**

**1.** Procédé pour la production d'esters (3-phtalidylidène) alkyliques de carnitine et d'alcanoylcarnitines de formule générale (I)

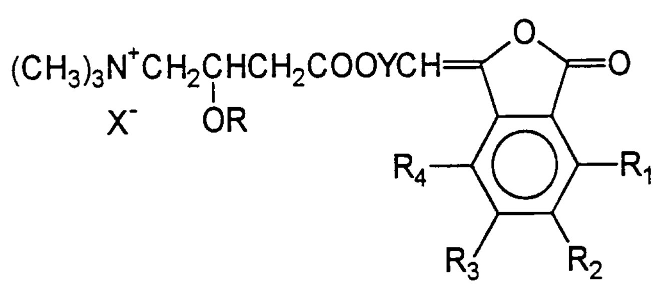

(I)

dans laquelle :

| | |
|---|---|
| Y | est un groupe alkylène en $C_1$-$C_5$ non substitué ou substitué avec un ou plusieurs groupes alkyle inférieurs en $C_1$-$C_4$ ; |
| R | est un atome d'hydrogène ou un groupe alcanoyle en $C_2$-$C_6$ ; |
| $R_1$, $R_2$, $R_3$ et $R_4$ | qui sont identiques ou différents, sont un atome d'hydrogène ou d'halogène ou un groupe alcoxy en $C_1$-$C_8$, alkyle inférieur en $C_1$-$C_4$, alkyle inférieur halogéno-substitué, amino, amino alkyle-substitué dont le groupe alkyle a 1 à 4 atomes de carbone, nitro, cyano ou alcanoylamino en $C_1$-$C_4$, ou $R_1$ et $R_2$ pris ensemble, $R_2$ et $R_3$ pris ensemble ou $R_3$ et $R_4$ pris ensemble forment un groupe alkylènedioxy en $C_1$-$C_4$, et $X^-$ est l'anion d'un sel pharmacologiquement acceptable, |

qui comprend la condensation de la carnitine ou d'une alcanoylcarnitine, dont le groupe alcanoyle a 2 à 6 atomes de carbone, avec un 3-(ω-bromoalkylidène)phtalide qui est facultativement substitué dans ses positions 4, 5, 6 et 7 avec respectivement les groupes $R_4$, $R_3$, $R_2$ et $R_1$ précédemment définis, en

présence de solvants dipolaires aprotiques, à une température de +5 à +80 °C, pendant des durées de réaction de 1 à 48 heures.